Europäisches Patentamt

(19) European Patent Office

Office européen des brevets

(11) Publication number: **0 108 517**
**B1**

(12) **EUROPEAN PATENT SPECIFICATION**

(45) Date of publication of patent specification: **10.10.90**

(51) Int. Cl.⁵: **A 61 K 7/08**

(21) Application number: **83306131.0**

(22) Date of filing: **11.10.83**

(54) Process for making shampoo compositions.

(30) Priority: **13.10.82 US 434026**
**14.09.83 US 532244**

(43) Date of publication of application:
**16.05.84 Bulletin 84/20**

(45) Publication of the grant of the patent:
**10.10.90 Bulletin 90/41**

(84) Designated Contracting States:
**AT BE CH DE FR IT LI NL SE**

(56) References cited:
**EP-A-0 034 846**　　**GB-A-1 051 268**
**DE-A-2 111 950**　　**US-A-3 476 489**
**FR-A-2 130 472**　　**US-A-4 185 106**

**SEIFEN-ÖLE-FETTE-WACHSE, vol. 101, no. 17,
1975, pages 507-508, CLR Chem. Laboratorium;
K. RICHTER: "Bioschwefel-Pulver und
Bioschwefel-Fluid"**

The file contains technical information
submitted after the application was filed and
not included in this specification

(73) Proprietor: **THE PROCTER & GAMBLE
COMPANY
301 East Sixth Street
Cincinnati Ohio 45201 (US)**

(72) Inventor: **Wahl, Errol Hoffman
8021 Deershadow Lane
Cincinnati Ohio 45242 (US)**

(74) Representative: **Brooks, Maxim Courtney et al
Procter & Gamble (NTC) Limited Whitley Road
Longbenton
Newcastle-upon-Tyne NE12 9TS (GB)**

Courier Press, Leamington Spa, England.

## EP 0 108 517 B1

**Description**

The present invention relates to a method for preparing antidandruff hair care compositions comprised of the water insoluble, zinc pyridinethione as particulate antidandruff agent, a suspending agent, an anionic surfactant and water, the compositions being substantially free of polymeric and clay type suspending agents.

The processing of antidandruff hair care compositions in general has been presented by several U.S. Patents, among which are US—A—3,476,489, November 4, 1969 to Mees and Olson and US—A—3,152,046, October 6, 1964 to Kaprol. GB—A—1,051,268, December 14, 1966 to Colgate-Palmolive, relates to a cold-blending process whereby selenium sulphide, having a particle size of 3 to 10 microns and being free of bentonite and sodium carboxymethylcellulose, is added with other components to form therapeutic hair care compositions. Seifen-Öle-Fette-Wachse, Vol. 101, No. 17, 507—508 (1975) and DE—A—2,111,950 describe processes for preparing creams and emulsion form shampoos in which powdered sulfur is admixed after cooling. US—A—4,185,106 relates to shampoo compositions based on hydroxypyridone antidandruff agents and prepared by cold blending.

The prior art also discloses processing hair care compositions containing zinc pyridinethione (ZPT) as an antidandruff agent. Of the references which do address such compositions, (eg: Gerstein, T., *J. Soc. Cosmet, Chem. 23*: 99—114 (1979)) none teach the processing method of the present invention.

It is an object, therefore, of the present invention to provide a processing method whereby the specific antidandruff agent particles are mixed with an anionic surfactant, a suspending agent and water to form highly effective antidandruff hair care compositions.

It is a further object of the present invention to not only provide effective antidandruff hair care compositions but to also provide aesthetically-pleasing compositions.

These and other objectives will become more apparent from the detailed description of the invention that follows. All percentages are by weight unless indicated to the contrary.

This invention relates to a process for preparing an antidandruff hair care composition comprising:

(a) heating from 50% to 90% of water to a temperature of from 71°C to 88°C;

(b) mixing with said 71°C to 88°C water from 2% to 30% of an anionic surfactant, optionally together with non-essential components of said composition;

(c) mixing with said water/anionic surfactant mixture from 1% to 7% of a nonpolymeric and nonclay suspending agent selected from ethylene glycol esters of fatty acids having from 16 to 22 carbon atoms, alkanolamides of fatty acids having from 16 to 22 carbon atoms, alkyl ($C_{16-22}$) dimethylamine oxides, and mixtures thereof;

(d) cooling said water/anionic surfactant/suspending agent mixture to a temperature of from 21°C to 49°C, achieving substantial crystallization of the suspending agent;

(e) adding from 0.05% to 2% of water insoluble, particulate antidandruff agent to said water/anionic surfactant/suspending agent mixture; and

(f) where the mixture of step (e) has a temperature in excess of 27°C, cooling the mixture to 27°C;

wherein said percentages are by weight of the composition prepared and said composition is substantially free of polymeric and clay type suspending agents, characterised in that the water-insoluble, particulate antidandruff agent of step (e) is zinc pyridinethione.

Most or all the components of the compositions, including the surfactant and the suspending agent but not the antidandruff agent, are mixed at temperatures ranging from 71°C. to 88°C. However, before adding the agent to the mixture, the temperature must be lowered to from 21°C. to 49°C. It is believed that this temperature reduction is necessary to result in minimal interference from materials such as the suspending agent and good deposition of the antidandruff agent onto the scalp.

The present process utilizes several essential components and may in addition utilize optional components.

## Essential components
### Antidandruff agent

The water insoluble, particulate antidandruff agent is present in the compositions made using the present process at a level of from 0.05% to 2%, preferably from 0.1% to 1.5%, most preferably from 0.25% to 1.25%. By water insoluble is meant having a solubility less than 0.01% at 25°C. The antidandruff agent useful herein is zinc pyridinethione. The particle size, mass median equivalent spherical diameter, of the agent can be of any desirable size but is preferably from 1 to 15 µm, most preferably 2 to 10 µm.

### Surfactants

The surfactants useful in the present invention can be any of a wide variety of anionic surfactants and are present at levels of from 2% to 30%, preferably from 7% to 20%, for a shampoo composition, made using the present process conditions. The term "surfactant" is intended to include soap although the synthetic surfactants are preferred.

Examples of suitable soaps are the sodium, potassium, ammonium and alkanol ammonium salts of higher fatty acids (those having 10—20 carbon atoms). Anionic nonsoap surfactants can be exemplified by the alkali metal salts of organic sulfuric reaction products having in their molecular structure an alkyl radical containing from 8—22 carbon atoms and a sulfonic acid or sulfuric acid ester radical (included in the term alkyl is the alkyl portion of higher acyl radicals). Olefin sulfonates are included as suitable surfactants herein. Preferred are the sodium, ammonium, potassium or triethanolamine alkyl sulfates, especially those obtained by sulfating the higher alcohols ($C_8$—$C_{18}$ carbon atoms), sodium coconut oil fatty acid monoglyceride sulfates and sulfonates; sodium or potassium salts of sulfuric acid esters of the reaction product of 1 mole of a high fatty alcohol (e.g., tallow or coconut oil alcohols) and 1 to 12 moles of ethylene oxide; sodium or potassium salts of alkyl phenol ethylene oxide ether sulfate with 1 to 10 units of ethylene oxide per molecule and in which the alkyl radicals contain from 8 to 12 carbon atoms; sodium alkyl glyceryl ether sulfonates; the reaction product of fatty acids having from 10 to 22 carbon atoms esterified with isethionic acid and neutralized with sodium hydroxide; water soluble salts of condensation products of fatty acids with sarcosine; and others known in the art. Alkyl sulfates, ethoxylated alkyl sulfates and mixtures thereof are preferred. Particularly preferred are ammonium alkyl sulfate, triethanolamine alkyl sulfate, sodium alkyl sulfate, sodium alkyl glyceryl ether sulfonates and mixtures thereof.

Suspending agent

The suspending agents useful in the present process can be any of a number of non-polymeric, nonclay materials. These materials include ethylene glycol esters of fatty acids having from 16 to 22 carbon atoms. Both ethylene glycol mono and distearate are examples of these esters.

Other useful suspending agents are alkanolamides of fatty acids having from 16 to 22 carbon atoms, preferably 16 to 18 carbon atoms. Suitable examples of these agents include stearic monoethanolamide, stearic diethanolamide, stearic monoisopropanolamide, stearic diethanolamide distearate and mixtures thereof.

Still other suitable suspending agents are alkyl ($C_{18}$—$C_{22}$) dimethylamine oxides such as stearyl dimethyl amine oxide. Another acceptable suspending agent is a mixture of glyceryl stearate and stearamidoethyl diethylamine. Mixtures of suspending agents are also acceptable. These suspending agents are present at levels of from 1% to 7%, preferably from 2% to 6%. These agents may also serve to give an unexpected pearlescence to the product when used to suspend the antidandruff particles of the present invention.

It is important in the present process that substantially all of the suspending agent be crystallized prior to the addition of the antidandruff agent. Thus, if the temperature to which the mixture minus the antidandruff agent is cooled is near the melting point of the suspending agent, the mixture must be held at that temperature for a long enough time for substantial crystallization to occur before the antidandruff agent is added. With ethylene glycol distearate, if the temperature is about 49°C., the mixture should be held for 30 minutes. Lower temperatures would not require such a holding period. Crystallization data may be obtained using a Differential Scanning Calorimeter (DSC) for example.

Water

The last essential component of the compositions made using the process of the present invention is water. The amount of water is from 50% to 90%, preferably from 75% to 85%.

Optional components

Compositions made using the present process can contain any of a wide variety of optional components. Such optional components are more fully discussed below.

Amide

An amide is a desirable optional component present in the compositions of the present process.

Any of the alkanolamides of fatty acids useful in shampoo compositions can be used. Generally, these include mono and diethanolamides of fatty acids having from 8 to 14 carbon atoms. Preferred compounds include coconut monoethanolamide, lauric or coconut diethanolamide, and mixtures thereof.

The amide is present at a level of from 2.0% to 4.0%. It is believed that a more rich lathering, more stable product results when amides of these types are combined with the other components of the invention at hand.

Conditioning aid

A desirable optional component for use herein is a conditioning agent such as propylene glycol or zwitterionics such as cocoamidopropyl betaine at a level of from 0.2% to 5%. These materials surprisingly interfere less than other agents such as fatty acids with the deposition of the antidandruff agent.

Others

Other ingredients useful in compositions made in accordance with the present invention include preservatives such as benzyl alcohol, methyl paraben, propyl paraben and imidozalidinyl urea. In addition, pH adjusters such as citric acid, sodium citrate, succinic acid, phosphoric acid, sodium hydroxide, sodium carbonate, triethanolamine, and mixtures thereof may be useful in the present invention. Other optional ingredients such as perfumes, dyes and coloring agents may also be added. These aesthetic agents as well as the others comprise, individually, from 0.1% to 2% of the composition if they are present.

3

Surfactants other than anionics can also be used as optional components in the process of the present invention. The preferred type of optional surfactants are nonionic and zwitterionic but cationic surfactants may also be used. The optional surfactants may be used at a level of from 0.1% to 10%, preferably 0.5% to 5%.

The pH of the compositions of the present process ranges from 3 to 9, preferably from about 3 to about 6. Proper pH is obtained through the use of an appropriate buffer system such as sodium citrate/citric acid. Other buffering systems are known to those knowledgeable in the art.

The compositions made according to the present invention are substantially free of polymeric and clay type suspending agents. Such agents include, for example, carboxymethylcellulose, hydroxypropyl guar gum, hydroxyethylcellulose, acrylic acid polymers, magnesium aluminum silicate and hydroxypropylmethylcellulose. It is believed that these suspending agents interfere with teh formation of a final product which possesses efficient antidandruff agent deposition. These should not be added as a separate component or with other materials such as the antidandruff agent. By substantially free is meant less than 0.02%. Not included as polymeric suspending agents herein are surfactants which may be polymeric.

### Method of manufacture

The method of the present invention begins by adding water to a mix tank and heating to from 71°C. to 88°C. Next, any surfactants, amides, suspending agents, buffers, perfumes, coloring agent, and preservatives are mixed at that temperature range. The batch is cooled to from 21°C. to 49°C., preferably from 21°C to 46°C., and the water insoluble particulate antidandruff agent then is added to the batch. This resulting mixture is preferably mixed until the antidandruff agent is well dispersed and is then cooled to 27°C.

The following example was prepared in accordance with the processing method of the present invention and is presented to further describe and demonstrate the invention described herein. This example is illustrative of the present invention.

### Example

The following shampoo composition was made using the process of the present invention:

| Component | Weight % |
|---|---|
| Zinc pyridinethione (48% active) | 2.08 |
| Triethanolamine alkyl sulfate (35% active) | 55.56 |
| Sodium chloride | 0.80 |
| Citric acid | 0.53 |
| Coconut monoethanolamide | 3.00 |
| Ethylene glycol distearate | 5.00 |
| Perfume | 0.60 |
| Color | 0.20 |
| Water | q.s. 100.00 |

This example was prepared by adding water to a mix tank and heating the water to 82°C. Next, citric acid and sodium chloride were added and mixed. The anionic surfactant, triethanolamine alkyl sulfate, was then mixed into the batch. Coconut monoethanolamine was mixed until it dissolved, as was the suspending agent, ethylene glycol distearate. The perfume, color and perservatives were added, and the batch was mixed for about 5 minutes.

This batch was cooled to a temperature of from 46°C. to 49°C. and the ZPT was added and mixed for 15 minutes. Finally, the entire batch was then cooled to 27°C.

### Claims

1. A process for preparing an antidandruff hair care composition comprising:

(a) heating from 50% to 90% of water to a temperature of from 71°C to 88°C;

(b) mixing with said 71°C to 88°C water from 2% to 30% of an anionic surfactant, optionally together with non-essential components of said composition;

(c) mixing with said water/anionic surfactant mixture from 1% to 7% of a nonpolymeric and nonclay suspending agent selected from ethylene glycol esters of fatty acids having from 16 to 22 carbon atoms, alkanolamides of fatty acids having from 16 to 22 carbon atoms, alkyl ($C_{16-22}$) dimethylamine oxides, and mixtures thereof;

(d) cooling said water/anionic surfactant/suspending agent mixture to a temperature of from 21°C to 49°C, achieving substantial crystallization of the suspending agent;

(e) adding from 0.05% to 2% of water insoluble, particulate antidandruff agent to said water/anionic surfactant/suspending agent mixture; and

(f) where the mixture of step (e) has a temperature in excess of 27°C, cooling the mixture to 27°C; wherein said percentages are by weight of the composition prepared and said composition is substantially free of polymeric and clay type suspending agents, characterized in that the water-insoluble, particulate antidandruff agent of step (e) is zinc pyridinethione.

2. A process according to Claim 1 characterized in that the temperature of step (d) is from 21°C to 46°C.

3. A process according to Claims 1 or 2 characterized in that the surfactant is selected from alkyl sulfates, ethoxylated alkyl sulfates, sodium alkyl glyceryl ether sulfonates, and mixtures thereof;

4. A process according to any of Claims 1 to 3 characterized in that the suspending agent is selected from ethylene glycol monostearate, ethylene glycol distearate, and mixtures thereof and is present at a level of from 2% to 5%.

5. A process according to any of Claims 1 to 4 characterized in that the surfactant is selected from sodium alkyl sulfate, triethanolamine alkyl sulfate, ammonium alkyl sulfate and mixtures thereof.

**Patentansprüche**

1. Verfahren zur Herstellung einer Antischuppen-Haarpflegezusammensetzung, umfassend:

(a) Erhitzen von 50% bis 90% Wasser auf eine Temperatur von 71°C bis 88°C;

(b) Mischen dieses Wassers von 71°C bis 88°C mit 2% bis 30% eines anionischen grenzflächenaktiven Mittels, wahlweise gemeinsam mit nicht-essentiellen Komponenten der genannten Zusammensetzung;

(c) Mischen dieses Gemisches aus Wasser/anionischem grenzflächenaktivem Mittel mit 1% bis 7% eines nicht-polymeren und nicht-tonartigen Suspendierungsmittels, ausgewählt unter Ethylenglykolestern von Fettsäuren mit 16 bis 22 Kohlenstoffatomen, Alkanolamiden von Fettsäuren mit 16 bis 22 Kohlenstoffatomen, Alkyl-($C_{16-22}$)-dimethylaminoxiden und Gemischen hievon;

(d) Kühlen des genannten Gemisches aus Wasser/anionischem grenzflächenaktivem Mittel/ Suspendierungsmittel auf eine Temperatur von 21°C bis 49°C, wobei wesentliche Kristallisation des Suspendierungsmittel erzielt wird;

(e) Zusetzen von 0,05% bis 2% wasserunlöslichem, partikulärem Antischuppenmittel zum genannten Gemisch aus Wasser/anionischem grenzflächenaktivem Mittel/Suspendierungsmittel; und

(f) wenn das Gemisch aus Schritt (e) eine Temperatur von mehr als 27°C aufweist, Kühlen des Gemisches auf 27°C;

worin die genannten Prozentsätze Gewichtsprozentsätze der hergestellten Zusammensetzung sind und die genannten Zusammensetzung im wesentlichen frei von polymeren und tonartigen Suspendierungsmitteln ist, dadurch gekennzeichnet, daß das wasserunlösliche, partikuläre Antischuppenmittel in Schritt (e) Zinkpyridinthion ist.

2. Verfahren nach Anspruch 1, dadurch gekennzeichnet, daß die Temperatur in Schritt (d) von 21°C bis 46°C beträgt.

3. Verfahren nach Anspruch 1 oder 2, dadurch gekennzeichnet, daß das grenzflächenaktive Mittel unter Alkylsulfaten, ethoxylierten Alkylsulfaten, Natriumalkylglycerylethersulfonaten und Gemischen hievon ausgewählt ist.

4. Verfahren nach einem der Ansprüche 1 bis 3, dadurch gekennzeichnet, daß das Suspendierungsmittel unter Ethylenglykolmonostearat, Ethylenglykoldistearat und Gemischen hievon ausgewählt ist und in einer Menge von 2% bis 5% vorliegt.

5. Verfahren nach einem der Ansprüche 1 bis 4, dadurch gekennzeichnet, daß das grenzflächenaktive Mittel unter Natriumalkylsulfat, Triethanolaminalkylsulfat, Ammoniumalkylsulfat und Gemischen hievon ausgewählt ist.

**Revendications**

1. Procédé de préparation d'une composition de soin des cheveux antipelliculaire comprenant:

(a) le chauffage de 50% à 90% d'eau jusqu'à une température de 71°C à 88°C;

(b) le mélange, avec cette eau à 71°C—88°C, de 2% à 30% d'un tensioactif anionique, éventuellement avec les constituants non essentiels de cette composition;

(c) le mélange avec ce mélange eau/tensioactif anionique de 1% à 7% d'un agent de suspension non polymère et non argileux, choisi parmi les esters d'éthylèneglycol d'acides gras comportant 16 à 22 atomes de carbone, les alcanolamides d'acides gras comportant de 16 à 22 atomes de carbone, les oxydes d'alkyl(en $C_{16}$—$C_{22}$)diméthylamines, et leurs mélanges;

(d) le refroidissement du mélange eau/tensioactif anionique/agent de suspension jusqu'à une température de 21°C à 49°C, pour aboutir essentiellement à la cristallisation de l'agent de suspension;

(e) l'addition de 0,05% à 2% d'agent antipelliculaire particulaire, insoluble dans l'eau, à ce mélange eau/tensioactif anionique/agent de suspension; et

(f) lorsque le mélange de l'étape (e) a une température dépassant 27°C, le refroidissement du mélange jusqu'à 27°C;

les pourcentages étant en poids de la composition préparée et ladite composition étant essentiellement exempte d'agents de suspension polymères ou de type argile, caractérisé en ce que l'agent antipelliculaire particulaire, insoluble dans l'eau, de l'étape (e) est la zinc-pyridinethione.

5

2. Procédé selon la revendication 1, caractérisé en ce que la température de l'étape (d) est de 21°C à 46°C.

3. Procédé selon la revendication 1 ou 2, caractérisé en ce que le tensioactif est choisi parmi les alkylsulfates, les alkylsulfates éthoxylés, les alkylglycéryléthersulfonates de sodium et leurs mélanges;

4. Procédé selon l'une quelconque des revendications 1 à 3, caractérisé en ce que l'agent de suspension est choisi parmi le monostéarate d'éthylèneglycol, le distéarate d'éthylèneglycol et leurs mélanges, et est présent en une proportion de 2% à 5%.

5. Procédé selon l'une quelconque des revendications 1 à 4, caractérisé en ce que le tensioactif est choisi parmi un alkylsulfate de sodium, un alkylsulfate de triéthanolamine, un alkylsulfate d'ammonium et leurs mélanges.